Europäisches Patentamt

(19) European Patent Office    (11) Publication number :    **0 229 786**
Office européen des brevets                             **B1**

(12)    EUROPEAN PATENT SPECIFICATION

(45) Date of publication of patent specification :    (51) Int. Cl.⁵ : **A 61 M 25/00**
28.03.90

(21) Application number : 86904055.0

(22) Date of filing : 18.07.86

(86) International application number :
PCT/BE 86/00025

(87) International publication number :
WO/87004 (29.01.87 Gazettee 87/03)

(54) **ANTIBACTERIAL CLOSURE SYSTEM.**

(30) Priority : 22.07.85 US 757586

(43) Date of publication of application :
29.07.87 Bulletin 87/31

(45) Publication of the grant of the patent :
28.03.90 Bulletin 90/13

(84) Designated contracting states :
BE DE FR GB IT

(56) References cited :
WO--A--83 /039 75
FR--A-- 2 493 149
NL--A-- 7 301 986
US--A-- 4 187 848
US--A-- 4 346 703

(73) Proprietor : BAXTER INTERNATIONAL INC. (a Dela-
ware corporation)
One Baxter Parkway
Deerfield Illinois 60015 (US)

(72) Inventor : PELUSO, Francesco
Verbindingslaan, 70
B-3030 Heverlee (BE)
Inventor : BALTEAU, Patrick
Rue du Fond du Ruisseau, 8
B-4240 Saint-Georges-sur-Meuse (BE)

(74) Representative : MacGregor, Gordon et al
ERIC POTTER & CLARKSON 14 Oxford Street
Nottingham, NG1 5BP (GB)

## Description

### Field of the invention

This invention relates to closure systems for medical tubing or for ports on medical apparatus. The invention particularly relates to closure systems which provide an antibacterial effect.

### Background of the invention

Medical procedures often require a connection where the bioburden (i. e., bacterial population) is minimized. Closure systems containing an antibacterial agent can reduce the bioburden by providing a bacteriocidal or bacteriostatic effect to the connection site prior to and after use. Closure systems having an antibacterial effect are particularly desirable for components used in peritoneal dialysis.

At the present time thousands of patients who have limited or nonexistent kidney function due to end state renal disease are being maintained by continuous ambulatory peritoneal dialysis (CAPD), along with other forms of peritoneal dialysis.

In the CAPD procedure, connections between dialysis solution containers and administration sets which communicate with the peritoneal catheter must be routinely made and broken, normally several times a day. Particularly when the patient is doing the CAPD exchanges alone, there is the possibility that the sterility of the flow path between the various solution containers and the peritoneal cavity may be compromised. Airborne bacteria or the accidental contamination of an open connector by the patient can contaminate the flow path. The result of such a contamination can be peritonitis.

Closure systems for medical connectors, such as CAPD connectors, have been developed.

For example, in the Quinton Cap manufactured by Quinton Instrument Co., a liquid antiseptic such as povidone iodine, is injected into the lumen of the catheter by means of a syringe and then covered with a cap.

As another example, FR-A-2 493 149 discloses an antiseptic end cap for a catheter. The end cap has a reservoir, in which a liquid antiseptic is retained. A movable wall can be displaced to force the liquid antiseptic from the reservoir into the bore of the catheter.

As yet another example, Genatempo et al U.S. Patent 4,440,207, which is assigned to the assignee of the present invention, discloses an end cap for a connector which is lined with an absorbent material containing an antiseptic.

FR-A-2 493 149 discloses a closure system comprising a connector including a luer fitment having an interior bore, a cap for joining to the connector and having an interior wall surface defining an interior chamber into which the luer fitment is advanced as the cap and connector are joined, and a liquid antiseptic retained in the chamber, a seal area being defined between the interior wall surface of the cap and the luer fitment, the seal being continuous as the luer fitment is advanced, whereby the liquid antiseptic is trapped in the chamber and directed therefrom into the interior bore of the luer fitment as the latter is advanced.

The present invention is characterised in that the connector has a skirt peripherally surrounding the luer fitment, and a lip is formed on the exterior wall surface of the cap, the lip continuously sealingly contacting the skirt as the luer fitment is advanced into the chamber.

### Brief description of the drawings

Fig. 1 is a plan view of a closure system which embodies the features of the invention and in which the associated connector and cap are joined together;

Fig. 2 is another view of the closure system shown in Fig. 1 with the cap removed and the connector joined to a second mating connector;

Fig. 3 is another view of the closure system shown in Fig. 2 after the mating connectors have been disconnected and prior to the attachment of a new cap;

Fig. 4 is an enlarged perspective view of the closure system shown in Fig. 1; and

Figs. 5 to 7 are side views, partly in section, of the closure system as the connector and cap are being progressively brought together.

Before explaining the embodiments of the invention in detail, it is to be understood that the invention is not limited in its application to the details of construction and the arrangement of components as set forth in the following description or as illustrated in the accompanying drawings. The invention is capable of other embodiments and of being practiced or carried out in various ways. Furthermore, it is to be understood that the phraseology and terminology employed is for the purpose of description and should not be regarded as limiting.

### Description of the preferred embodiments

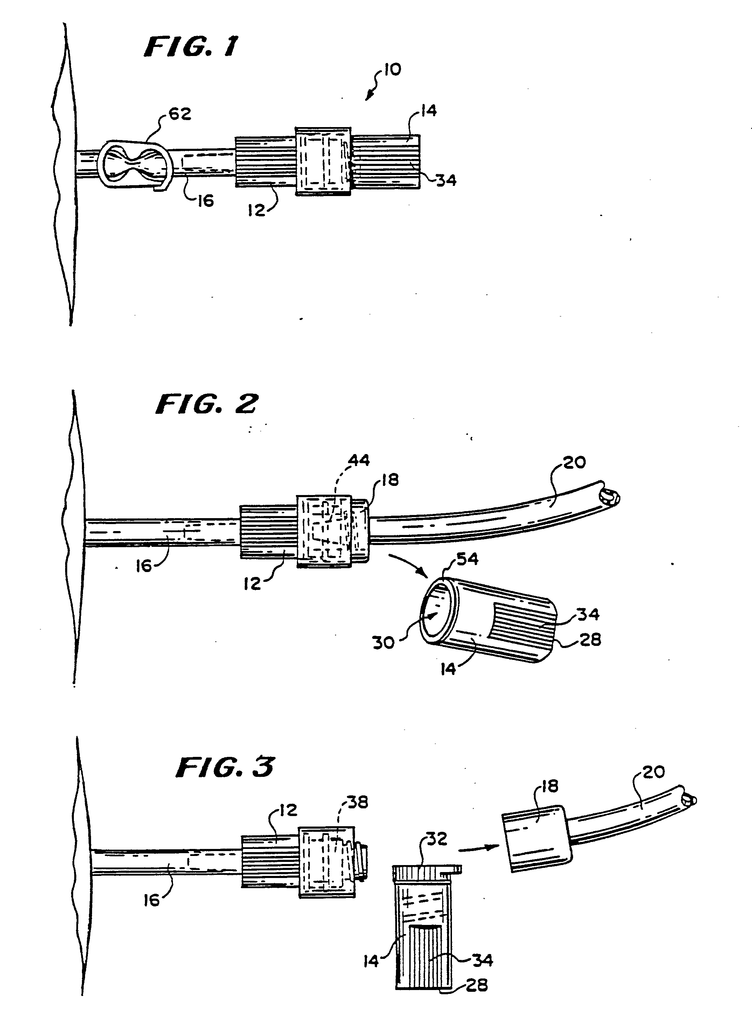

A closure system 10 which embodies the features of the invention is shown in Fig. 1. The system 10 includes a luer-type connector 12 and a removable protective cap 14 for the connector 12.

The system 10 can be used in different operative environments. In the illustrated embodiment, the system 10 is used in conjunction with a peritoneal dialysis procedure. In this modality, the connector 12 is carried at the end of a catheter 16 which communicates with the peritoneal cavity of a patient. An extension set (not shown) can be used to interconnect the connector 12 with the end of the catheter 16. The cap 14 not only serves to protect the interior portions of the connector 12 from touch contact, but it also serves to provide an active antibacterial effect to prevent contamination of the connector 12 and adjacent portions of the catheter 16 or extension set. The closure system 10 thus reduces the potential for peritonitis, which is always a matter of paramount concern in peritoneal dialysis.

In use, the protective cap 14 is removed to expose the connector 12, when desired. This is shown in Fig. 2. The connector 12 can then be conveniently attached to suitable mating connector 18. As shown in Fig. 2, the mating connector 18 is carried at the end of a fluid administration set 20.

Once the resulting connection has been made between the connectors 12 and 18, spent peritoneal dialysis solution can be conducted out of the peritoneal cavity of the patient. Fresh peritoneal dialysis solution can then be conducted back into the peritoneal cavity so that the peritoneal dialysis process can continue. Upon the introduction of fresh dialysis solution, the two connectors 12 and 18 are separated, breaking the connection. This is shown in Fig. 3.

A new protective cap 14 is now attached to the connector 12. The protective cap 14 remains on the connector 12 (as shown in Fig. 1) while the just-freshly introduced peritoneal solution dwells within the peritoneal cavity for a predetermined period, typically about six hours. During this dwell period, the patient is free to carry on his or her activities, with the closure system 10 unobtrusively hidden beneath his or her clothing.

At the end of the dwell period, the cap 14 is removed and, preferably, discarded. Another exchange of spent-for-fresh peritoneal solution is made in the manner just described.

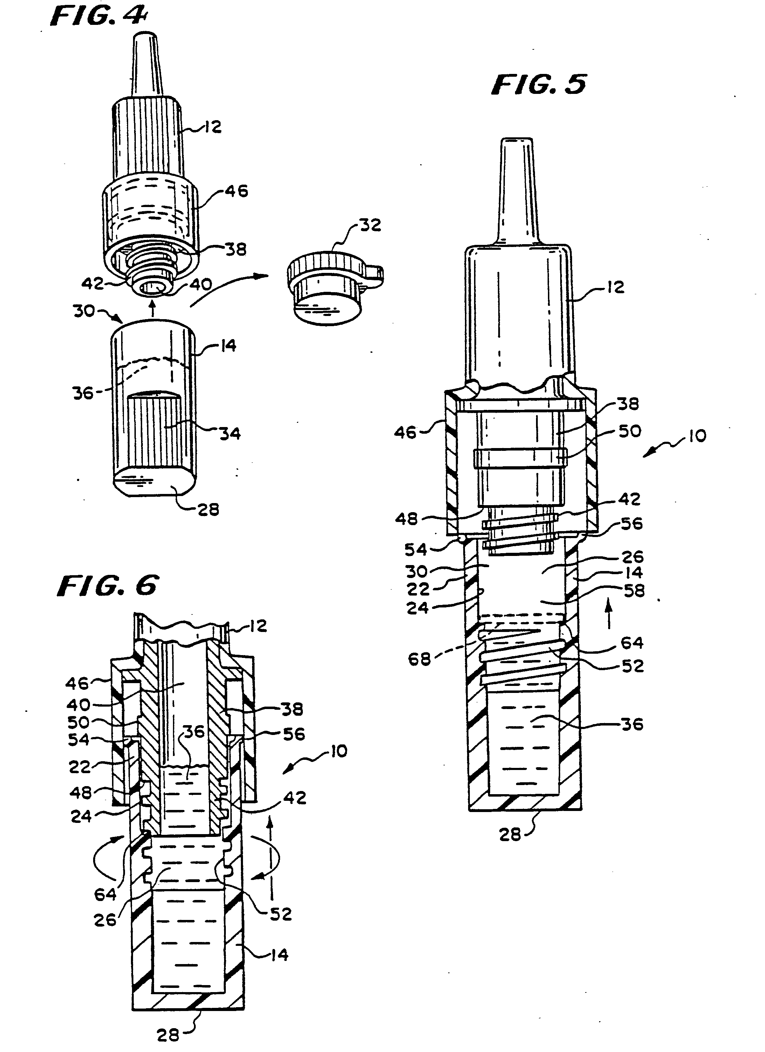

As best shown in Figs. 4 and 5, the protective cap 14 comprises a generally cylindrical body having an exterior wall surface 22 and an interior wall surface 24. The interior wall surface 24 defines an interior chamber 26. The chamber 26 has a closed end 28 and an open end 30. As shown in Fig. 3, the open end 30 is sealed prior to use by a removable plug 32.

The exterior wall surface 22 of the cap 14 may include a flattened, roughened portion 34 to facilitate the patient's grip on the cap 14 during its attachment and removal from the connector 12.

A liquid antiseptic 36 is carried within the cap chamber 26 to provide an active bacteriocidal effect when the cap 14 is attached to the connector 12. Prior to use, the plug 32 retains the antiseptic 36 within the chamber 26.

As is also best shown in Figs. 4 and 5, the connector 12 includes a luer fitment 38 and an annular skirt 46 which peripherally surrounds the fitment 38. While the luer fitment 38 may vary, in the illustrated embodiment, the fitment 38 comprises a female luer having a standard tapered interior bore 40. In the illustrated embodiment, the fitment 38 also includes a threaded distal end 42.

In this arrangement, the mating connector 18 comprises a suitably threaded male luer fitment 44 (shown in phantom lines in Fig. 1) which threadably engages the threaded distal end 42 within the skirt 46 and which sealingly occupies the bore 40 of the female luer fitment 38.

As best shown in Fig. 5, the threaded distal end 42 of the luer fitment 38 has an outer diameter which is less than the outer diameter of the rest of the fitment 38. An annular shoulder 48 is thus created on the fitment 38 near the threaded distal end 42.

Near this annular shoulder 48 there is also an upstanding ridge 50 formed on the fitment 38. This ridge 50 has a further increased outer diameter, compared to the rest of the fitment 38.

As can also be seen in Fig. 5, the interior cap wall surface 24 includes an interior threaded portion 52. This threaded portion 52 mates with the threaded distal end 42 of the luer fitment 38. The inner diameter of the threaded portion 52 is generally equal to the outer diameter of the threaded distal end 42 of the fitment 38.

An annular lip 54 extends outwardly from the exterior cap wall surface 22 at the open end 30 of the cap 14. As shown in Fig. 5, the lip 54 contacts the interior of the skirt 46, there forming a first sealed area 56.

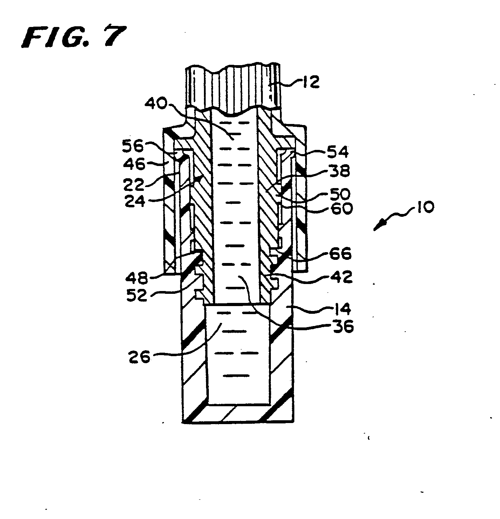

As can be seen in Figs. 6 and 7, as the luer fitment 38 is progressively advanced into the interior chamber 26 of the cap 14, the first sealed area 56 formed between the annular lip 54 and the interior of the skirt 46 is continuously maintained.

As shown in Fig. 5, the region 58 of the cap 14 between its threaded portion 52 and its open end 30 has a larger inner diameter than the inner diameter of the threaded portion 52. This larger inner diameter is generally equal to the outer diameter of the ridge 50 formed on the fitment 38. Thus, as the luer fitment is progressively advanced into the interior chamber 26, a second sealed area 60 is formed between this cap region 58 and the ridge 50.

Also, as can be seen in Figs. 6 and 7, as the fitment 38 is progressively advanced into the cap chamber 26, this second sealed area 60, like the heretofore described first sealed area 56, is continuously maintained.

By continuously maintaining the first and second sealed areas 56 and 60 as the luer fitment 38 is progressively threaded onto the cap 14, leakage of the liquid antiseptic 36 from the cap chamber 26 is prevented.

Since leakage is prevented, the antiseptic is effectively trapped within the cap chamber 26. Thus, as the threaded distal end 42 of the fitment is progressively advanced, the incompressible liquid antiseptic 36 trapped in the cap chamber 26 is directed out of the cavity 26 and into the bore 40 of the fitment 38. This is shown in Figs. 6 and 7.

When the cap 14 is fully threaded upon the connector 12 (see Fig. 7), the liquid antiseptic 36 occupies substantially all of the bore 40 of the fitment 38 and the adjacent portion of the extension set on catheter 16. As shown in Fig. 1, a clamp 62 is used to prevent the undesired transport of either the peritoneal dialysis solution out of the peritoneal cavity or the antiseptic 36 into the peritoneal cavity.

Because the inner diameter of the cap 14 in the region 68 is larger than the inner diameter in the threaded portion 52, an internal shoulder 64 is formed within the cap chamber 26. As shown in Fig. 7, when the cap 14 is fully threaded upon the luer fitment 38, the fitment shoulder 48 seats against the cap shoulder 64.

Together, the first and second sealed areas 56 and 60 effectively trap the antiseptic 36 within the confines of the cap chamber 26 as the fitment 38 is advanced to occupy more and more of the chamber 26. As a result, the antiseptic 36 has no place to escape other than into the bore 40 of the fitment 38.

Thereafter, when the cap 14 is completely attached to the connector 12, the first and second sealed areas 56 and 60 prevent leakage of the antiseptic 36 from the closure system 10 during use. Upon separation of the cap 14 from the connector 12, the skirt 46 serves to collect drops of antiseptic which may escape as the double seal arrangement is broken during separation, thereby preventing contact between the antiseptic and the user.

While, in the illustrated embodiment, the connector and the cap 14 are threaded together, the invention is operative in other configurations. For example, the luer fitment 38 can take the form of a spike member, and the cap 14 can be placed onto and off of the spike with a push-pull motion. As in the illustrated embodiment, by providing two seal areas between the spike and cap, the antiseptic can be trapped within the cap chamber 26 as the spike is inserted therein. The antiseptic will be directed onto the lumen of the spike as the spike and cap are joined, just as the antiseptic is directed into the bore 40 of the fitment 38 in the illustrated configuration.

An alternate embodiment of the cap 14 is shown in phantom lines in Fig. 5. There, the cap 14 includes a frangible membrane 68 which seals the antiseptic 36 within the cap chamber 26.

In this arrangement, because the antiseptic 36 is sealed within the cap chamber 26 by the membrane 68, a separate plug 32 need not be used.

As the threaded distal end 42 of the fitment 38 is advanced into the chamber 26, the frangible membrane 68 is broken by the fitment end 42. The liquid antiseptic 36 is then directed into the bore 40 of the fitment 38 in the manner heretofore described.

The components of the closure system 10 can be made of various materials, depending upon the operative environment in which they are used. In the context of peritoneal dialysis, the cap 14 is made of a suitable thermoplastic material which is capable of being sterilized and which is inert to the liquid antiseptic used. Antiseptics which may be used include sodium chloride and povidone iodine. The luer fitment 38 of the connector 12 can likewise be made of a sterilizable and inert thermoplastic material. Alternately a sterilizable and inert metallic material may be used, such as titanium or stainless steel. Preferably, the remainder of the connector 12 is made of a sterilizable and inert thermoplastic material.

**Claims**

1. A closure system comprising a connector (12) including a luer fitment (38) having an interior bore, a cap (14) for joining to the connector and having an interior wall surface (24) defining an interior chamber (26) into which the luer fitment (38) is advanced as the cap and connector are joined, and a liquid antiseptic (36) retained in the chamber (26), a seal area (60) being defined between the interior wall surface (24) of the cap and the luer fitment (38), the seal being continuous as the luer fitment is advanced, whereby the liquid antiseptic (36) is trapped in the chamber (26) and directed therefrom into the interior bore of the luer fitment as the latter is advanced, characterised in that the connector (12) has a skirt (46) peripherally surrounding the luer fitment (38), and a lip (54) is formed on the exterior wall surface (22) of the cap, the lip continuously sealingly contacting the skirt as the luer fitment is advanced into the chamber (26).

2. A closure system according to claim 1, wherein the luer fitment (38) includes an external threaded portion (42), and the interior wall surface (24) includes a threaded portion (52) which threadably engages the threaded portion of the luer fitment as the luer fitment is advanced into the interior chamber (26).

3. A closure system according to claim 1 or 2, wherein the seal area (60) between the interior wall surface (24) of the cap and the luer fitment (38) is defined by a ridge (50) formed on the luer fitment and sealingly contacting the interior wall surface (24).

4. A closure system according to claim 1, 2 or 3, wherein the cap includes a removable plug member (32) for sealing said interior chamber (26) prior to use.

5. A closure system according to claim 1, 2 or 3, wherein the cap includes a membrane (68) disposed within the interior chamber (26) of the cap for normally sealing the antiseptic (36) within the chamber and the membrane being breakable by the luer fitment to open the interior chamber, as the luer fitment is advanced into the chamber.

**Patentansprüche**

1. Verschlußsystem, umfassend: einen Verbinder (12), der ein Luer-Anschlußstück (38) mit einer Innenbohrung aufweist, eine Kappe (14), die an den Verbinder anschließbar ist und eine Innenwandfläche (24) aufweist, die eine Innenkammer (26) begrenzt, in die beim Zusammenfügen der Kappe und des Verbinders das Luer-Anschlußstück (38) eingeführt wird, und ein in der Kammer (26) befindliches flüssiges Antiseptikum (36) wobei ein Dichtbereich (60) zwischen der Innenwandfläche (24) der Kappe und dem Luer-Anschlußstück (38) gebildet ist und die Dichtung während des Vorschubs des Luer-Anschlußstücks kontinuierlich ist, wodurch das flüssige Antiseptikum (36) in der Kammer (26) eingeschlossen und während des Vorschubs des Luer-Anschlußstücks von dort in die Innenbohrung desselben gerichtet wird, dadurch gekennzeichnet, daß der Verbinder (12) einen Mantel (46) aufweist, der das Luer-Anschlußstück (38) umfangsmäßig umgibt, und eine Lippe (54) an der Außenwandfläche (22) der Kappe gebildet ist, wobei die Lippe während des Vorschubs des Luer-Anschlußstücks in die Kammer (26) den Mantel kontinuierlich dichtend kontaktiert.

2. Verschlußsystem nach Anspruch 1, wobei das Luer-Anschlußstück (38) einen Außengewindeabschnitt (42) und die Innenwandfläche (24) einen Gewindeabschnitt (52) aufweist, der während des Vorschubs des Luer-Anschlußstücks in die Innenkammer (26) mit dem Gewindeabschnitt des Luer-Anschlußstücks verschraubt wird.

3. Verschlußsystem nach Anspruch 1 oder 2, wobei der Dichtbereich (60) zwischen der Innenwandfläche (24) der Kappe und dem Luer-Anschlußstück (38) von einer Rippe (50) gebildet ist, die auf dem Luer-Anschlußstück geformt ist und die Innenwandfläche (24) dichtend kontaktiert.

4. Verschlußsystem nach Anspruch 1, 2 oder 3, wobei die Kappe einen abnehmbaren Stöpsel (32) aufweist, der die Innenkammer (26) vor Gebrauch dicht verschließt.

5. Verschlußsystem nach Anspruch 1, 2 oder 3, wobei die Kappe eine in ihrer Innenkammer (26) angeordnete Membran (68) aufweist, die das Antiseptikum (36) in der Kammer normalerweise dicht abschließt, wobei die Membran während des Vorschubs des Luer-Anschlußstücks in die Kammer durch das Luer-Anschlußstück durchstoßbar ist, um die Innenkammer zu öffnen.

**Revendications**

1. Système de fermeture comprenant un connecteur (12) qui comporte un raccord de type luer (38) à passage intérieur, un capuchon (14) prévu pour être assemblé avec le connecteur et présentant une surface de paroi intérieure (24) qui définit une chambre intérieure (26) dans laquelle le raccord luer (38) pénètre pendant l'assemblage du capuchon et du connecteur, et un antiseptique liquide (36) retenu dans la chambre (26), une zone d'étanchéité (60) étant définie entre la surface de paroi intérieure (24) du capuchon et le raccord luer (38), l'étanchéité étant permanente pendant la pénétration du raccord luer, de sorte que l'antiseptique liquide (36) est emprisonné dans la chambre (26) et dirigé, à partir de celle-ci, dans le passage intérieur du raccord luer lorsque ce dernier pénètre, caractérisé en ce que le connecteur (12) comporte une jupe (46) entourant périphériquement le raccord luer (38), et en ce qu'une lèvre (54) est formée sur la surface de paroi extérieure (22) du capuchon, la lèvre étant en contact d'étanchéité permanent avec la jupe lorsque le raccord luer pénètre dans la chambre (26).

2. Système de fermeture suivant la revendication 1, dans lequel le raccord luer (38) comprend une partie filetée extérieurement (42), et la surface de paroi intérieure (24) comprend une partie filetée (52) dans laquelle se visse la partie filetée du raccord luer lorsque le raccord luer pénètre dans la chambre intérieure (26).

3. Système de fermeture suivant la revendication 1 ou 2, dans lequel la zone d'étanchéité (60) entre la surface de paroi intérieure (24) du capuchon et le raccord luer (38) est définie par une nervure (50) prévue sur le raccord luer et qui vient en contact étanche avec la surface de paroi intérieure (24).

4. Système de fermeture suivant la revendication 1, 2 ou 3, dans lequel le capuchon comporte un bouchon amovible (32) pour obturer ladite chambre intérieure (26) avant l'utilisation.

5. Système de fermeture suivant la revendication 1, 2 ou 3, dans lequel le capuchon comprend une membrane (68) disposée dans la chambre intérieure (26) du capuchon pour retenir normalement de façon étanche l'antiseptique (36) à l'intérieur de la chambre, la membrane pouvant être brisée par le raccord luer de manière à ouvrir la chambre intérieure lorsque le raccord luer pénètre dans la chambre.

*FIG. 1*

*FIG. 2*

*FIG. 3*

**FIG. 4**

12
46
38
42 40
32
30
14
36
34
28

**FIG. 5**

12
46
38
50
10
48
42
54
56
30
26
22
14
24
58
68
64
52
36
28

**FIG. 6**

12
46
40
50
54
22
48
24
36
38
56
10
42
64
26
52
14
28

FIG. 7